# EUROPÄISCHE PATENTSCHRIFT

(11) **EP 1 082 942 B1**
(45) Veröffentlichungstag und Bekanntmachung des Hinweises auf die Patenterteilung: **02.08.2006**
(21) Anmeldenummer: 99117842.7
(22) Anmeldetag: 10.09.1999
(51) Int. Cl.: A61B 17/50, A61D 1/00

(54) **Vorrichtung zum Entfernen von Zecken**
Device for removing ticks
Dispositif pour l'enlevement des ticques

(43) Veröffentlichungstag der Anmeldung: 14.03.2001
(73) Patentinhaber: Dr. Schick Gmbh, 74889 Sinsheim (DE)
(72) Erfinder: Schick, Gerhard, Dr., 74889 Sinsheim (DE)
(74) Vertreter: Naumann, Ulrich

(56) Entgegenhaltungen:
- WO-A-99/04712
- DE-U- 29 500 134

## Beschreibung

Die Erfindung betrifft eine Vorrichtung zum Entfernen von Zecken mit einer zangenähnlichen Greifeinrichtung.

Vorrichtungen der in Rede stehenden Art sind seit langem aus der Praxis bekannt und existieren in den unterschiedlichsten Formen und Größen. Dabei sind die Vorrichtungen häufig in Form von Pinzetten ausgebildet. Derartige Vorrichtungen finden ihre Verwendung sowohl im privaten als auch im gewerblichen Bereich immer dann, wenn Zecken von der menschlichen oder tierischen Haut während des Blutsaugens der Zecken zu entfernen sind. Eine solche Entfernung von Zecken von der Haut ist erforderlich, da durch den Stich der Zecken gefährliche Krankheiten auf Mensch und Haustiere übertragen werden können. Sowohl beim Entfernen mittels der Finger als auch mittels einer Pinzette läßt sich meist nur der beim Saugakt stark anschwellende Zeckenkörper abreißen, während die Mundwerkzeuge der Zecke nach wie vor in der Haut stecken bleiben. Die Übertragung von Krankheiten ist in diesem Zustand nach wie vor möglich.

Eine vollständige Entfernung einer Zecke während des Blutsaugens läßt sich meist nur durch ein gefühlvolles Greifen bzw. Festhalten und anschließendes Herausdrehen der Mundwerkzeuge der Zecke erreichen. Die Drehbewegung bewirkt dabei ein vorsichtiges Ziehen der Zecke. Außerdem wird durch das Drehen eine Stabilisierung zwischen Mundwerkzeugen und Zeckenkörper erreicht, so daß mit der bekannten Vorrichtung in fast allen Fällen ein Abreißen der Mundwerkzeuge verhindert werden kann.

Bei der bekannten Vorrichtung ist jedoch problematisch, daß trotz des Ergreifens der Zecke mit einer speziellen Greifeinrichtung ein Zerquetschen und Zerplatzen des vollgesogenen Zeckenkörpers nicht immer vermeidbar ist. Die Gefahr des Zerquetschens und Zerplatzens des Zeckenkörpers besteht insbesondere im stark voligesogenen Zustand des Zeckenkörpers. Anschließend ist eine sichere und saubere Entfernung der Zecke nicht mehr gewährleistet.

Das Dokument DE 295 00 134 U offenbart eine zangenähnliche Greifeinrichtung.

Eine Aufnahmeeinrichtung ist aus WO 99/04712 A bekannt.

Der vorliegenden Erfindung liegt daher die Aufgabe zugrunde, eine Vorrichtung zum Entfernen von Zecken der eingangs genannten Art anzugeben, bei der ein problemloses Entfernen von Zecken von der Haut auch bei stark vollgesogenem Zeckenkörper bei einfachster Handhabung ermöglicht ist.

Erfindungsgemäß wird die voranstehende Aufgabe durch die Merkmale des Patentanspruchs 1 gelöst. Danach ist die in Rede stehende Vorrichtung durch eine eine Ausnehmung aufweisende Aufnahmeeinrichtung zur Aufnahme einer Zecke gekennzeichnet.

In erfindungsgemäßer Weise ist zunächst erkannt worden, daß eine herkömmliche, zangenähnliche Greifeinrichtung für eine Zecke zum Entfernen vollgesogener Zecken nicht immer optimal geeignet ist. Dabei ist eine Abkehr von der herkömmlichen Greifeinrichtung bzw. eine Ergänzung der Greifeinrichtung vorgenommen worden. Hierzu ist die Vorrichtung in weiter erfindungsgemäßer Weise durch eine Aufnahmeeinrichtung zur Aufnahme einer Zecke ergänzt worden, die eine Ausnehmung aufweist. Mit der Aufnahmeeinrichtung und genauergesagt mit der Ausnehmung ist es möglich, eine Zecke einfach aufzunehmen und herauszudrehen bzw. herauszuziehen, ohne daß durch eine Greifbewegung bzw. ein zangenähnliches Zupacken mit sich aufeinander zu bewegenden Greifelementen die Gefahr des Zerquetschens des Zeckenkörpers besteht. Dabei kann die Zecke einfach zwischen Mundwerkzeugen und Zeckenkörper aufgenommen werden.

Durch die Ausgestaltung der Vorrichtung mit sowohl einer Greifeinrichtung als auch einer Aufnahmeeinrichtung ist eine Vorrichtung realisiert, die in kombinierter Weise sowohl für noch nicht sehr stark vollgesogene oder noch gar nicht vollgesogene Zeckenkörper als auch für sehr stark vollgesogene Zeckenkörper immer die geeignete Entfernungseinrichtung bietet.

Folglich ist mit der erfindungsgemäßen Vorrichtung zum Entfernen von Zecken eine Vorrichtung angegeben, bei der ein problemloses Entfernen von Zecken von der Haut auch bei stark vollgesogenem Zeckenkörper bei einfachster Handhabung ermöglicht ist.

Im Hinblick auf eine besonders einfache Aufnahme einer Zecke könnte die Ausnehmung in einem flächigen Bereich der Aufnahmeeinrichtung ausgebildet sein. Zur Aufnahme könnte die Vorrichtung dann mit dem flächigen Bereich auf eine entsprechende flächige Hautpartie aufgesetzt werden und könnte die Aufnahmeeinrichtung auf der Hautpartie derart verschoben werden, bis die zu entfernende Zecke in der Ausnehmung zur Anlage mit der Aufnahmeeinrichtung gelangt. Hierauf könnte ein Herausdrehen oder Herausziehen der Zecke erfolgen.

Zur Gewährleistung eines besonders sicheren Aufnehmens der Zecke im Bereich der Mundwerkzeuge oder zwischen den Mundwerkzeugen und dem Zeckenkörper könnte der flächige Bereich zumindest im Bereich der Ausnehmung konvex oder im wesentlichen gewölbt ausgebildet sein. Hierdurch ist ein sicheres Gleiten des Bereichs der Ausnehmung unmittelbar auf der Haut gesichert. Mit anderen Worten ist der flächige Bereich - ausgehend vom Bereich der Ausnehmung - von der Haut, auf die die Aufnahmeeinrichtung aufgesetzt ist, weggewölbt.

Hinsichtlich eines besonders einfachen und sicheren Herausziehens der Zecke könnte der flächige Bereich im der Ausnehmung entgegengesetzten Bereich eine Abschrägung aufweisen.

Im Hinblick auf ein sicheres Aufnehmen der Zecke könnte die Ausnehmung zwei aufeinander zulaufende Wandungen aufweisen. Die Aufnahme der Zecke könnte dann vorzugsweise im engsten Bereich der Ausnehmung erfolgen. Dabei ist von Vorteil, wenn die Wandungen in einem spitzen Winkel aufeinander zulaufen, so daß während des Aufnehmens der Zecke möglichst schnell ein Eingriff zwischen der Aufnahmeeinrichtung und der Zecke zustande kommt.

In einer konstruktiv besonders einfachen Ausgestaltung könnte die Ausnehmung im wesentlichen dreiecksförmig ausgebildet sein. Dabei wären geradlinige Wandungen realisiert, die besonders einfach herstellbar sind.

Die Wandungen könnten hinsichtlich eines besonders sicheren Aufnehmens der Zecke derart abgeschrägt sein, daß sie einander im wesentlichen gegenüberliegende, spitzwinklige Aufnahmebereiche bilden. Dabei könnte eine Abschrägung gegen die Ebene eines flächigen Bereichs der Aufnahmeeinrichtung erfolgen.

Zur Vermeidung eines ungewollten Verkippens oder gar Abreißens des Zeckenkörpers während seiner Aufnahme könnten die Wandungen in ihrem Kontaktbereich derart abgeschrägt sein, daß sie stetig ineinander übergehen. Des weiteren könnten die Wandungen entlang ihrer gesamten Länge, einschließlich des Kontaktbereichs, im selben Winkel abgeschrägt sein. Dieser Winkel könnte 45° betragen. Somit ist ein sicheres Entfernen einer Zecke ermöglicht.

In einer konstruktiv besonders einfachen und optisch ansprechenden Ausgestaltung könnte der flächige Bereich als eine die Ausnehmung aufweisende Grundfläche einer im wesentlichen kreiszylinderförmigen Aufnahmeeinrichtung ausgebildet sein. Zur Gewährleistung eines möglichst großen und damit sicheren Aufnahmebereichs könnte die Ausnehmung über den Kreismittelpunkt der Grundfläche hinaus ausgebildet sein. Dabei könnte die Ausnehmung im Sinne eines schlanken Spalts ausgebildet sein.

Weiterhin im Hinblick auf eine sichere Aufnahme der Zecke bzw. des Zeckenkörpers könnte der Zylindermantel im Bereich der Öffnung der Ausnehmung einen Durchgang aufweisen. Die Aufnahmeeinrichtung könnte dann derart zur Zecke hin bewegt werden, daß die Zecke durch die Öffnung der Ausnehmung in die Ausnehmung hinein bewegt wird. Dabei tritt die Zecke gleichzeitig durch den Durchgang des Zylindermantels in das Innere des Zylinders ein. Damit ist die Zecke teilweise vom Zylinder umschlossen.

Zur Gewährleistung einer sicheren Aufnahme auch von sehr großen, d.h. sehr stark vollgesogenen Zecken, könnte der Durchgang in etwa entlang eines halben Kreisbogens der Zylindergrundfläche ausgebildet sein. Dabei wäre im wesentlichen der maximale Durchgang bezüglich einer vorgegebenen Zylindergröße realisiert.

In konstruktiv besonders einfacher Weise könnte die Aufnahmeeinrichtung dem dem Greifbereich der Greifeinrichtung abgewandten Ende der Greifeinrichtung zugeordnet sein. Somit wäre eine Vorrichtung realisiert, die an einem Ende eine Greifeinrichtung und am anderen Ende eine Aufnahmeeinrichtung aufweist.

In weiter konstruktiv besonders einfacher Weise könnte die Aufnahmeeinrichtung mit der Greifeinrichtung verbunden sein. Dabei ist insbesondere eine integrale Ausbildung mit der Greifeinrichtung vorteilhaft. Hierzu könnte die Aufnahmeeinrichtung entweder mit der Greifeinrichtung vergossen oder gemeinsam mit der Greifeinrichtung spritzgußtechnisch hergestellt sein. Als Material könnte jeder gut verarbeitbare Kunststoff verwendet werden.

Zur Gewährleistung einer einfachen Handhabung der Vorrichtung könnte die Greifeinrichtung und/oder die Aufnahmeeinrichtung zumindest teilweise von einem Griffstück umgeben sein. Dabei könnte das Griffstück in konstruktiv besonders einfacher Weise als Hülse ausgebildet sein. Eine derartige Hülse könnte aus Kunststoff oder in einer besonders stabilen Ausführung aus Metall hergestellt sein.

Bei einer Ausführung des Griffstücks als Hülse könnten die Greifeinrichtung an einem Ende der Hülse und die Aufnahmeeinrichtung am anderen Ende der Hülse angeordnet sein. In einer besonders praktischen Ausführung der Vorrichtung könnte die Greifeinrichtung durch Drücken auf die Aufnahmeeinrichtung betätigbar sein. Hierbei wäre wiederum die Ausgestaltung der Vorrichtung mit einer Hülse von Vorteil, da das Betätigen der Greifeinrichtung durch Daumendruck auf die Aufnahmeeinrichtung bei gleichzeitigem Halten der Vorrichtung in einer Hand erfolgen könnte. Hierbei könnte die Greifeinrichtung zum Betätigen ihrer Greifelemente mit der Hülse wechselwirken.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im allgemeinen bevorzugte Ausgestaltungen und Weiterbildungen der Lehre erläutert. In der Zeichnung zeigen
- Fig. 1: in einer Seitenansicht, schematisch, das Ausführungsbeispiel einer erfindungsgemäßen Vorrichtung,
- Fig. 2: in vier Seitenansichten, um jeweils 90° gedreht, das Ausführungsbeispiel einer erfindungsgemäßen Vorrichtung,
- Fig. 3: in einer Seitenansicht, vergrößert, die Aufnahmeeinrichtung des Ausführungsbeispiels gemäß Fig. 2 und
- Fig. 4: in einer geschnittenen Darstellung entlang der Linie IV-IV die Aufnahmeeinrichtung gemäß Fig. 3.

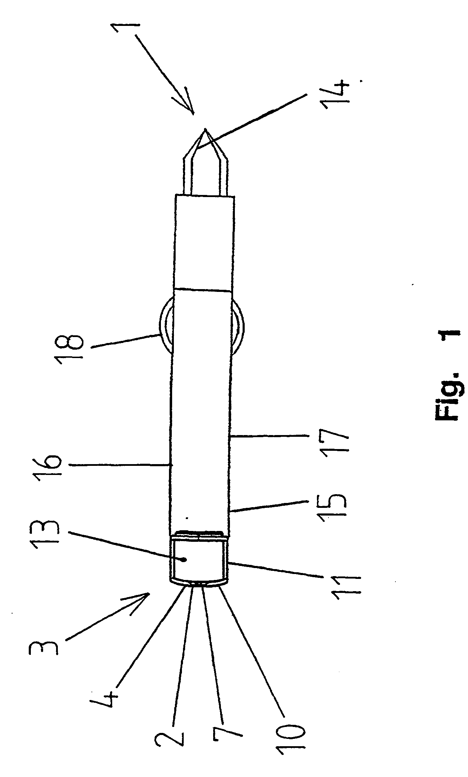

Fig. 1 zeigt in einer schematischen Seitenansicht das Ausführungsbeispiel einer erfindungsgemäßen Vorrichtung zum Entfernen von Zecken. Die Vorrichtung weist eine zangenähnliche Greifeinrichtung 1 auf. Am anderen Ende weist die Vorrichtung eine Aufnahmeeinrichtung 3 mit einer Ausnehmung 2 zur Aufnahme einer Zecke auf. Die Aufnahmeeinrichtung 3 ermöglicht ein problemloses Entfernen von Zecken von der Haut auch bei stark vollgesogenem Zeckenkörper, ohne daß die Gefahr für ein Zerquetschen oder Zerplatzen des Zeckenkörpers besteht.

Zur Handhabung der Vorrichtung ist die Greifeinrichtung 1 teilweise von einem Griffstück 16 umgeben. Das Griffstück 16 ist als Hülse 17 ausgebildet, die das zum Entfernen der Zecke günstige Drehen des Zeckenkörpers erleichtert.

Die Greifeinrichtung 1 ist über Betätigungselemente 18 betätigbar. Alternativ oder zusätzlich hierzu könnte die Greifeinrichtung 1 durch Drücken auf die Aufnahmeeinrichtung 3 betätigbar sein. Dabei würde die Greifeinrichtung 1 in der Hülse 17 verschoben werden.

Die Aufnahmeeinrichtung 3 ist genauergesagt an dem dem Greifbereich 14 der Greifeinrichtung 1 abgewandten Ende 15 der Greifeinrichtung 1 angeordnet.

Die Ausnehmung 2 ist in einem flächigen Bereich 4 der Aufnahmeeinrichtung 3 ausgebildet. Des weiteren umfaßt die Ausnehmung 2 zwei aufeinander zulaufende Wandungen 7.

Der flächige Bereich 4 ist als eine die Aufnehmung 2 aufweisende Grundfläche 10 einer im wesentlichen kreiszylinderförmigen Aufnahmeeinrichtung 3 ausgebildet. Der Zylindermantel 11 weist im Bereich der Öffnung 12 in der Ausnehmung 2 einen Durchgang 13 zur Aufnahme eines Zeckenkörpers auf.

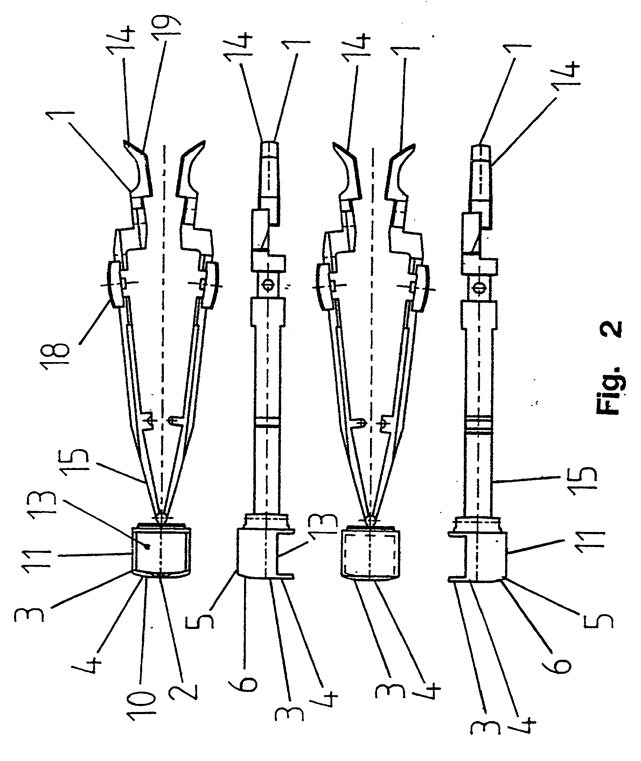

Fig. 2 zeigt in vier jeweils um 90° gedrehten Seitenansichten das Ausführungsbeispiel einer erfindungsgemäßen Vorrichtung zum Entfernen von Zecken. Die Drehung erfolgt dabei um die Längsachse der Greifeinrichtung 1. Aus Gründen der Übersichtlichkeit ist die Hülse 17 weggelassen. Die Aufnahmeeinrichtung 3 ist mit der Greifeinrichtung 1 integral verbunden.

In der obersten Darstellung der Fig. 2 ist die Ausnehmung 2 besonders gut erkennbar. Sowohl in der zweiten als auch in der vierten Darstellung von oben ist erkennbar, daß der flächige Bereich 4 im der Ausnehmung 2 entgegengesetzten Bereich 5 eine Abschrägung 6 aufweist. Diese Abschrägung 6 ist beim Herausziehen der Zecke besonders günstig, da die Aufnahmeeinrichtung 3 bzw. der flächige Bereich 4 besonders problemlos über die Haut des Menschen oder des Tieres gleiten kann.

In Fig. 2 ist die zylinderförmige Ausgestaltung der Aufnahmeeinrichtung 3 mit einer Grundfläche 10 und einem Zylindermantel 11 besonders gut erkennbar. Des weiteren ist der Aufbau der Greifeinrichtung 1 mit den Betätigungselementen 18 im Detail erkennbar.

In der zweiten und vierten Darstellung von oben ist jeweils nur das in Blickrichtung obere Greifelement 19 dargestellt.

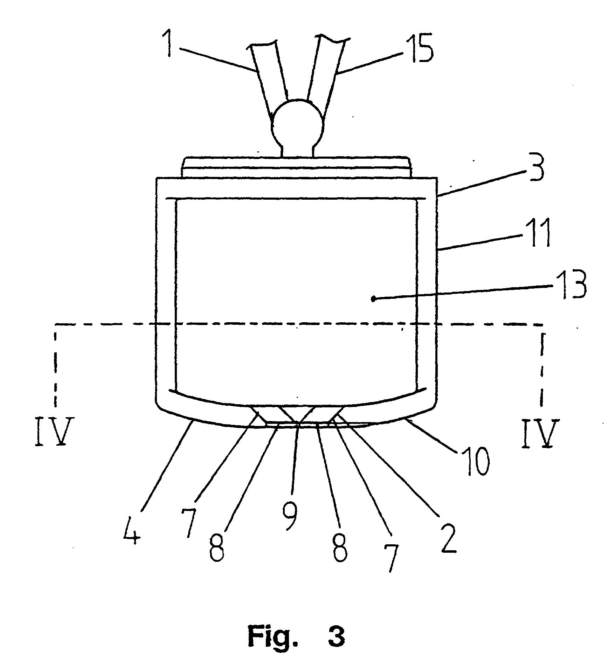

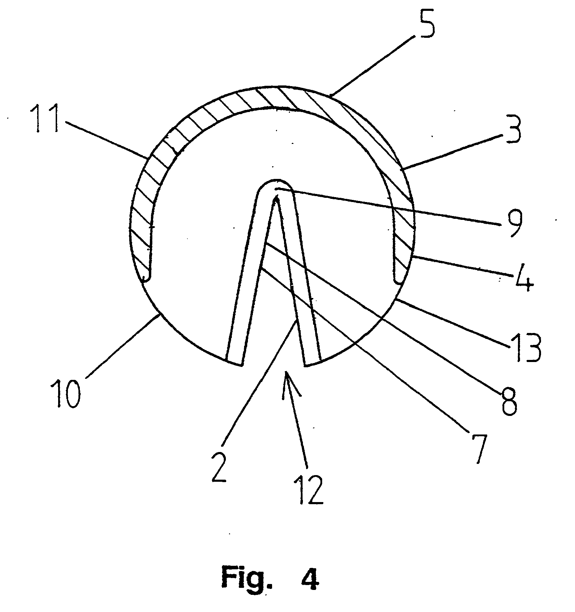

Die Figuren 3 und 4 zeigen in einer vergrößerten Seitenansicht sowie in einer geschnittenen Darstellung entlang der Linie IV-IV aus Fig. 3 die Aufnahmeeinrichtung 3 des Ausführungsbeispiels gemäß Fig. 2. Die Aufnahmeeinrichtung 3 weist eine im wesentlichen V-förmige oder dreiecksförmige Ausnehmung 2 auf. Die Ausnehmung 2 ist in einem flächigen Bereich 4 ausgebildet, der zumindest im Bereich der Ausnehmung 2 konvex oder im wesentlichen gewölbt ausgebildet ist.

Die zwei aufeinander zulaufenden Wandungen 7 sind derart abgeschrägt, daß sie einander im wesentlichen gegenüberliegende, spitzwinklige Aufnahmebereiche 8 bilden. Die Wandungen 7 sind in ihrem Kontaktbereich 9 derart abgeschrägt, daß sie stetig ineinander übergehen. Des weiteren sind die Wandungen 7 entlang ihrer gesamten Länge, einschließlich des Kontaktbereichs 9, im selben Winkel von etwa 45° abgeschrägt.

Die Ausnehmung 2 ist über den Kreismittelpunkt der Grundfläche 10 hinaus ausgebildet. Der Zylindermantel 11 weist im Bereich der Öffnung 12 der Ausnehmung 2 einen Durchgang 13 auf. Mit anderen Worten verläuft der Zylindermantel 11 nicht entlang der gesamten Grundfläche 10. Der Durchgang 13 ist etwa entlang eines halben Kreisbogens ausgebildet.

Hinsichtlich weiterer vorteilhafter Ausgestaltungen der Vorrichtung zum Entfernen von Zecken wird zur Vermeidung von Wiederholungen auf den allgemeinen Teil der Beschreibung sowie auf die nachgeordneten Patentansprüche verwiesen.

Abschließend sei hervorgehoben, daß das voranstehend erörterte Ausführungsbeispiel der erfindungsgemäßen Vorrichtung lediglich zur Erörterung der beanspruchten Lehre dient, diese jedoch nicht auf das Ausführungsbeispiel einschränkt.

## Patentansprüche

1. Vorrichtung zum Entfernen von Zecken, mit einer zangenähnlichen Greifeinrichtung (1), wobei die Greifeinrichtung (1) einen Greifbereich (14) aufweist und zumindest teilweise von einem Griffstück (16) umgeben ist,
**dadurch gekennzeichnet, dass** eine Aufnahmeeinrichtung (3) dem dem Greifbereich (14) der Greifeinrichtung (1) abgewandten Ende (15) der Greifeinrichtung (1) zugeordnet ist und dass die Aufnahmeeinrichtung (3) in etwa den Querschnitt des Griffstücks (16) hat.

2. Vorrichtung nach Anspruch 1, **dadurch gekennzeichnet, dass** die Ausnehmung (2) in einem flächigen Bereich (4) der Aufnahmeeinrichtung (3) ausgebildet ist, wobei der flächige Bereich (4) zumindest im Bereich der Ausnehmung (2) konvex oder im wesentlichen gewölbt ausgebildet sein kann und im der Ausnehmung (2) entgegengesetzten Bereich (5) vorzugsweise eine Abschrägung (6) aufweist.

3. Vorrichtung nach Anspruch 1 oder 2, **dadurch gekennzeichnet, dass** die Ausnehmung (2) im wesentlichen dreiecksförmig ausgebildet ist und vorzugsweise zwei aufeinander zu laufende Wandungen (7) aufweist, wobei die Wandungen (7) in einem spitzen Winkel aufeinander zulaufen.

4. Vorrichtung nach Anspruch 3, **dadurch gekennzeichnet, dass** die Wandungen (7) derart abgeschrägt sind, dass sie einander im wesentlichen gegenüberliegende, spitzwinklige Aufnahmebereiche (8) bilden.

5. Vorrichtung nach Anspruch 3 oder 4, **dadurch gekennzeichnet, dass** die Wandungen (7) in ihrem Kontaktbereich (9) derart abgeschrägt sind, dass sie stetig ineinander übergehen.

6. Vorrichtung nach einem der Ansprüche 3 bis 5, **dadurch gekennzeichnet, dass** die Wandungen (7) entlang ihrer gesamten Länge, einschließlich des Kontaktbereichs (9), im selben Winkel abgeschrägt sind, wobei der Winkel vorzugsweise 45 Grad beträgt.

7. Vorrichtung nach einem der Ansprüche 2 bis 6, **dadurch gekennzeichnet, dass** der flächige Bereich (4) als eine die Ausnehmung (2) aufweisende Grundfläche (10) einer im wesentlichen kreiszylinderförmigen Aufnahmeeinrichtung (3) ausgebildet ist, wobei die Ausnehmung (2) über den Kreismittelpunkt der Grundfläche (10) hinaus ausgebildet sein kann, wobei der Zylindermantel (11) im Bereich der Öffnung (12) der Ausnehmung (2) einen Durchgang (13) aufweisen kann und wobei der Durchgang (13) in etwa entlang eines halben Kreisbogens ausgebildet sein kann.

8. Vorrichtung nach einem der Ansprüche 1 bis 7, **dadurch gekennzeichnet, dass** die Aufnahmeeinrichtung (3) mit der Greifeinrichtung (1) verbunden ist.

9. Vorrichtung nach einem der Ansprüche 1 bis 8, **dadurch gekennzeichnet, dass** die Aufnahmeeinrichtung (3) integral mit der Greifeinrichtung (1) ausgebildet ist.

10. Vorrichtung nach einem der Ansprüche 1 bis 9, **dadurch gekennzeichnet, dass** die Aufnahmeeinrichtung (3) mit der Greifeinrichtung (1) vergossen oder gemeinsam spritzgußtechnisch hergestellt ist.

11. Vorrichtung nach einem der Ansprüche 1 bis 10, **dadurch gekennzeichnet, dass** die Greifeinrichtung (1) und/oder die Aufnahmeeinrichtung (3) zumindest teilweise von einem Griffstück (16) umgeben ist, wobei das Griffstück (16) als Hülse (17) ausgebildet ist.

12. Vorrichtung nach einem der Ansprüche 1 bis 11, **dadurch gekennzeichnet, dass** die Greifeinrichtung (1) durch Drücken auf die Aufnahmeeinrichtung (3) betätigbar ist.

## Claims

1. Device for removing ticks, having a pincer-like gripping device (1), the gripping device (1) having a gripping region (14) and being at least partially surrounded by a grip piece (16),
**characterised in that** a receiving device (3) is associated with the end (15) of the gripping device (1) that is remote from the gripping region (14) of the gripping device (1) and **in that** the receiving device (3) substantially has the cross-sectional area of the grip piece (16).

2. Device according to claim 1, **characterised in that** the recess (2) is constructed in a planar region (4) of the receiving device (3), the planar region (4) being able to be constructed so as to be convex or substantially curved at least in the region of the recess (2) and preferably having a bevelled portion (6) in the region (5) that is opposite the recess (2).

3. Device according to claim 1 or 2, **characterised in that** the recess (2) is constructed in a substantially triangular manner and preferably has two walls (7) which extend towards each other, the walls (7) extending towards each other at an acute angle.

4. Device according to claim 3, **characterised in that** the walls (7) are bevelled in such a manner that they form acutely angled receiving regions (8) which are arranged substantially opposite each other.

5. Device according to claim 3 or 4, **characterised in that** the walls (7) are bevelled in the contact region (9) thereof in such a manner that they merge with each other in a continuous manner.

6. Device according to any one of claims 3 to 5,
**characterised in that** the walls (7) are bevelled at the same angle along the entire length thereof, including the contact region (9), the angle preferably being 45 degrees.

7. Device according to any one of claims 2 to 6,
**characterised in that** the planar region (4) is constructed as a base face (10) of a substantially circular-cylindrical receiving device (3), which base face (10) has the recess (2), the recess (2) being able to be constructed so as to extend beyond the circular centre of the base face (10), the outer cylinder surface (11) being able to have an opening (13) in the region of the opening (12) of the recess (2) and the opening (13) being able to be constructed along a substantially semi-circular arc.

8. Device according to any one of claims 1 to 7,
**characterised in that** the receiving device (3) is connected to the gripping device (1).

9. Device according to any one of claims 1 to 8,
**characterised in that** the receiving device (3) is constructed integrally with the gripping device (1).

10. Device according to any one of claims 1 to 9,
**characterised in that** the receiving device (3) is cast with the gripping device (1) or is produced together with it using an injection moulding technique.

11. Device according to any one of claims 1 to 10,
**characterised in that** the gripping device (1) and/or the receiving device (3) is at least partially surrounded by a grip piece (16), the grip piece (16) being in the form of a sleeve (17).

12. Device according to any one of claims 1 to 11,
**characterised in that** the gripping device (1) can be activated by pressing on the receiving device (3).

## Revendications

1. Dispositif pour l'enlèvement des tiques, avec un dispositif de prise (1) analogue à une pince, le dispositif de prise (1) présentant une zone de prise (14) et étant entouré au moins partiellement par une poignée (16),
**caractérisé par le fait qu'**un dispositif de ramassage (3) est adjoint à l'extrémité (15) du dispositif de prise (1) écartée de la zone de prise (14) du dispositif de prise (1) et que le dispositif de ramassage (3) a approximativement la section transversale de la poignée (16).

2. Dispositif selon la revendication 1,
**caractérisé par le fait que** l'évidement (2) est formé dans une zone mince (4) du dispositif de ramassage (3), la zone mince (4) pouvant être réalisée convexe ou pour l'essentiel courbe au moins dans la zone de l'évidement (2) et présentant de préférence un biseau (6) dans la zone (5) opposée à l'évidement (2).

3. Dispositif selon la revendication 1 ou 2,
**caractérisé par le fait que** l'évidement (2) est formé pour l'essentiel triangulaire et présente de préférence deux parois (7) dirigées l'une vers l'autre, les parois (7) étant dirigées l'une vers l'autre selon un angle aigu.

4. Dispositif selon la revendication 3,
**caractérisé par le fait que** les parois (7) sont biseautées de telle manière qu'elles forment des zones de ramassage (8) à angle aigu pour l'essentiel opposées.

5. Dispositif selon la revendication 3 ou 4,
**caractérisé par le fait que** les parois (7) sont biseautées dans leur zone de contact (9) de telle manière qu'elles se convertissent de manière continue l'une dans l'autre.

6. Dispositif selon l'une des revendications 3 à 5,
**caractérisé par le fait que** les parois (7) sont biseautées selon le même angle sur toute leur longueur, y compris de la zone de contact (9), l'angle étant de préférence égal à 45 degrés.

7. Dispositif selon l'une des revendications 2 à 6,
**caractérisée par le fait que** la zone mince (4) est formé selon une surface (10) présentant l'évidement (2) d'un dispositif de ramassage (3) pour l'essentiel de forme cylindrique circulaire, l'évidement (2) pouvant être formé dépassant le centre de la surface (10), l'enveloppe cylindrique (11) pouvant présenter dans la zone de l'ouverture (12) de l'évidement (2) un passage (13) et le passage (13) pouvant être formé le long d'à peu près une moitié d'arc de cercle.

8. Dispositif selon l'une des revendications 1 à 7,
**caractérisé par le fait que** le dispositif de ramassage (3) est relié au dispositif de prise (1).

9. Dispositif selon l'une des revendications 1 à 8,
**caractérisé par le fait que** le dispositif de ramassage (3) est formé d'une seule pièce avec le dispositif de prise (1).

10. Dispositif selon l'une des revendications 1 à 9,
**caractérisé par le fait que** le dispositif de ramassage (3) est coulé avec le dispositif de prise (1) ou fabriqué en même temps par une technique de moulage par injection.

11. Dispositif selon l'une des revendications 1 à 10,
**caractérisé par le fait que** le dispositif de prise (1) et/ou le dispositif de ramassage (3) sont entourés au moins partiellement par une poignée (16), la poignée (16) étant réalisée sous forme d'une douille (17).

12. Dispositif selon l'une des revendications 1 à 11,
**caractérisé par le fait que** le dispositif de prise (1) peut être actionné par pression sur le dispositif de ramassage (3).
